# EUROPEAN PATENT APPLICATION

(11) **EP 4 552 549 A1**
(43) Date of publication of application: **14.05.2025**
(21) Application number: 23209346.8
(22) Date of filing: 13.11.2023
(51) Int. Cl.: A61B 3/10, A61B 3/12, A61B 5/00, G01J 3/02, G01J 3/28, G01B 9/02091

(54) **A DEVICE FOR MEASURING THE CHARACTERISTICS OF THE HUMAN EYE USING OCT TECHNOLOGY**

(71) Applicant: OPTOPOL Technology Spolka z ograniczona Odpowiedzialnoscia, 42-400 Zawiercie (PL)
(72) Inventor: Bednarczyk, Grzegorz, 42-400 Zawiercie (PL); Slusarczyk, Grzegorz, 40-213 Katowice (PL); Staniszewski, Pawel, 42-480 Poreba (PL)
(74) Representative: AOMB Polska Sp. z.o.o.

(57) **Abstract**

This invention concerns a device for measuring the characteristics of the human eye with the OCT technology, used for measuring eye properties, that has the following components within the optical path of the light beam:
- a light beam source with a low time coherence,
- a light beamsplitter used for dividing a single light beam coming from the light beam source into two and directing them towards the tested eye and the reference path and for directing the light beams that return from the reference path and from the tested eye, which enables the interference of those returning light beams and directing them further to the appropriate spectrometer,
- optical elements of the reference path and optical elements for directing the light onto the tested eye,
- an appropriate spectrometer comprising:
- a collimator,
- a diffraction element,
- an optical lens,
- a detector,

characterised in that the device is provided with additional optical means configured and suited for adjusting the spectrometer's imaging window by adjusting the spectral range of the wavelength recorded by the detector, which means are located within the spectrometer.

## Description

The object of the invention is a device for measuring the characteristics of the human eye using the optical coherence tomography (OCT) technology.

In prior art, there are many devices designed for testing the condition of the human eye structures, which use light beams to provide insight into various parts of the eye.

In prior art, there are known devices, the so-called OCTs (optical coherence tomographs), that have their optical system organised in such a way that the light beams can interact with particular types of eye structures, for example the retina or cornea. Such devices are calibrated so that they can provide good sharpness and accuracy of measurement for predefined and limited measuring coordinates at x-y-z axes. If such devices are suitable for measuring one part of the eye, be it the cornea or the retina, then the other part cannot be measured without any modifications, since both parts are located more than ten millimetres away (with such a distance, even the commonly used image sharpening systems cannot compensate for the differences in the extremely varied characteristics of light beam refraction, etc.).

On the other hand, prior art knows solutions that enable testing both areas of the human eye referred to above by means of a single, more complex optical system. One of such solutions is known from document EP4027861A1 where, in order to switch the operating mode (between scanning of the posterior part of the eye/retina and scanning of the anterior part of the eye/cornea area), we change the position of individual optical elements in steps (one of the lenses is shifted or introduced to / removed from the optical path, etc.) to shift the plane for focusing scanner beams in the human eye between posterior and anterior part of eye.

Nevertheless, even those devices that make it possible to switch the measuring mode between the anterior and posterior parts of the eye have a predefined height of imaging window that is restricted by equipment capability. They can measure the posterior part of the eye by positioning the imaging plane at a specific depth of the eye and maintaining the sharpness, and thus detect the eye's superficial structure within +/- 1 mm from that plane. The limitations arise from many years of experience, measurements and expected shapes of eye structures, and the most common expected deformations.

In prior art, there are known such solutions that enable us, to some extent, to increase the imaging window of OCTs. The SD-OCT method uses a light source with a low time coherence and a spectrometer for measuring the interference signal. Today's SSOCTs and SD-OCTs can double the imaging window by:
- introducing a phase shift between neighbouring A-scans by means of a piezoelectric actuator (PZT), electro-optical phase modulator and acoustic-optical modulator, scanning the sample axis shift, and a reference arm mirror;
- using dispersion in the optical system;
- displaying a decoupled image based on an image recognised by an image recognition algorithm that only leaves the recognised image on the screen (the so-called Full Range methods).

The Full Range method is offered by the commercially available devices by OPTOPOL Technology Sp. z o. o. as well as in OPTOVUE Solix by Visionix, Huvitz HOCT-1/1F, Cirrus 5000, Cirrus 6000 by Carl Zeiss Metitec AG.

The described Full Range methods have the following disadvantages:
- phase methods are sensitive to the longitudinal motion of the object (the so-called motion artifacts) that hinder the diagnosis;
- a visible artifact taking the form of a line around the zero frequency; - a long computing time (these methods are iterative);
- considerably reduced nominal scanning rate;
- the imaging window can only be doubled.

Unfortunately, in the case of very serious retina pathologies or elements of the eye chamber pathologies (for instance, a heavily swollen optic nerve), it turns out that the imaging window limitations existing in OCTs are too narrow to make a proper and reliable measurement (and the auto-focus systems are not suitable for compensating for such big differences in the height of the existing structures). The areas that go beyond the imaging window will thus be completely omitted in the measuring results or, even if they are included, their measurements will be disturbed.

Therefore, the presently known SD-OCT (Spectral Domain Optical Coherence Tomography, generally called OCT) solutions lack the variable depth of the imaging window that would provide considerable freedom as to the scope of measurement and ensure high accuracy and a relatively short time of measurement. The operator, when testing an object, in particular the human eye (retina or cornea), cannot adjust the depth of the imaging window to the high level of pathology of the retina or the tested front eye chamber. Moreover, many of the existing OCT systems and devices are highly sensitive to longitudinal motions of the eyes (called motion artifacts) that may occur during testing; even though we know methods that compensate for such interference by means of software and, possibly, repeated measurements, which considerably reduce the effective rate of eye scanning.

The aim of this invention is, therefore, to improve the human eye testing devices (OCTs) in such a way as to enable precise measurement of an eye structure even with serious pathologies / deformations of particular eye structures while maintaining a high rate of measurement.

That aim is achieved with a device for measuring the characteristics of the human eye in the OCT technology, used for measuring eye properties, that has the following components within the optical path of the light beam:
- a light beam source with a low time coherence,
- a light beamsplitter used for dividing a single light beam coming from the light beam source into two and directing them towards the tested eye and the reference path, and for directing the light beams that return from the reference path and from the tested eye, which enables the interference of those returning light beams and directing them further to the appropriate spectrometer,
- optical elements of the reference path and optical elements for directing the light onto the tested eye,
- an appropriate spectrometer comprising:
   - a collimator,
   - a diffraction element,
   - an optical lens,
   - a detector,
characterised in that the device is provided with additional optical means configured and suited for adjusting the spectrometer's imaging window by adjusting the spectral range of wavelength recorded by the detector, which means are located within the spectrometer.

Preferably, the additional optical means are selected from a group including a set of replaceable optical lenses, a deformable multifocal lens, means for shifting the optical lens relative to the detector, means for shifting one of the lenses in the optical lens, and an additional rotating diffraction element, or a combination thereof.

Preferably, the additional optical means have the form of a set of optical lenses that replace a single optical lens, each of which is suitable for providing a different imaging window height.

Preferably, the device has two to five sets of optical lenses.

Preferably, the device has three sets of optical lenses.

Preferably, the additional optical means have the form of a deformable multifocal lens being part of the spectrometer's optical lens, which is accompanied by means for deforming the multifocal lens.

Preferably, the additional optical means have the form of means for shifting the position of the optical lens relative to the detector.

Preferably, the additional optical means have the form of means for shifting one of the lenses of the optical lens.

Preferably, the additional optical means have the form of two sets of optical lenses, each of which is provided with means for shifting one of the lenses.

Preferably, the additional optical means have the form of the second diffraction element located between the diffraction element and the optical lens, and the second diffraction element is provided with means for shifting its position by changing its position angle.

Preferably, the first diffraction element is also provided with means for changing its position by changing its position angle.

Preferably, the device is additionally fitted with a movable focal lens for switching the light beam focusing area between the posterior part of the eye (i.e., the area of the retina) and the anterior chamber of the eye (i.e., the area of the cornea and lens).

The methods based on image detection are sensitive to the quality of layer recognition, which causes artifacts that hinder diagnosis. The proposed solution does not have the said disadvantages and is simple to implement, but even so, it is not limited to being used as the only solution; it can be used together with the Full Range methods described above.

### Brief description of the drawings:

Fig. 1 shows schematically the optical system of a spectrometer provided with a set of optical lenses, which uses one of them with a particular height of the imaging window,
Fig. 2 shows schematically the optical system of the spectrometer from Fig. 1 using a different optical lens with a different height of the imaging window than that in Fig. 1,
Fig. 3 shows schematically the optical system of a spectrometer provided with a multifocal lens in one configuration, with a particular height of the imaging window,
Fig. 4 shows schematically the optical system of the spectrometer from Fig. 3 in a different configuration of the multifocal length resulting in a different height of the imaging window than in Fig. 3,
Fig. 5 shows schematically the optical system of a spectrometer provided with means for shifting the optical lens, with the optical lens in one position and with a particular height of the imaging window,
Fig. 6 shows schematically the optical system of the spectrometer from Fig. 5 with the optical lens in a different position and with a different height of the imaging window than in Fig. 5,
Fig. 7 shows schematically the optical system of a spectrometer with an optical lens provided with a movable lens, with one position of that lens and with a particular height of the imaging window,
Fig. 8 shows schematically the optical system of the spectrometer from Fig. 7 with the movable lens in a different position and with a different height of the imaging window than in Fig. 7,
Fig. 9 shows schematically the optical system of a spectrometer provided with two different optical lenses, each of which also has a movable lens,
Fig. 10 shows schematically the optical system of a spectrometer provided with an additional diffraction element that can change its position by changing the position angle,
Fig. 11 shows schematically the optical system of a spectrometer provided with an additional diffraction element where both diffraction elements can change their positions by changing the position angle,
Fig. 12 shows schematically the elements of the device for measuring the characteristics of the human eye in the OCT technology, provided additionally with a movable lens (3) for switching the measuring mode between the anterior and the posterior parts of the eye and with a spectrometer provided with two optical lenses, each with a different focal length and different imaging windows.

### List of numerical symbols:

1 - output from the optical fibre
2 - collimator
3 - (first) diffraction element / transmission grid
4 - optical lens
5 - detector/camera
6 - the second optical lens
7 - the third optical lens
8 - schematic direction of optical lenses switching
9 - movable multifocal lens
10 - schematic direction of longitudinal shift of the optical lens
11 - schematic direction of longitudinal shift of one of the lenses of the optical lens
12 - the second diffraction element, e.g., a reflection grid
13 - schematic direction of change in the angular position of the diffraction element / diffraction elements relative to one another
14 - optical elements providing sharpness / autofocus
15 - optical interfaces
16 - optical path of the reference beam of the OCT tomograph
17 - optical divider / connector / coupler
22 - optical path of the object beam of the OCT tomograph
23 - schematic direction of introducing to / removing from the optical path an additional lens for switching the tomograph operating mode between the anterior and the posterior parts of the eye
S_OCT - OCT spectrometer

### Description of preferable embodiments of the invention:

This invention will now be presented in detail in the preferred embodiments, in an illustrative manner and without limitation.

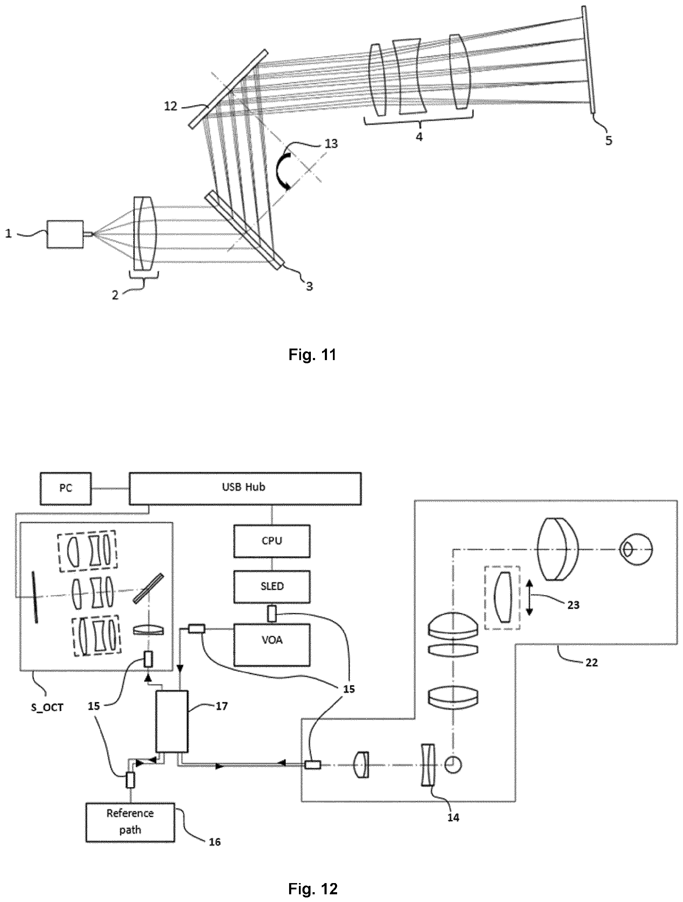

This invention directly relates to the field of optical coherence tomographs (OCTs) for scanning human eye structures 12, and its general design is shown in Fig. 12. Typically, the electronic and computer-based elements of an OCT are presented schematically by the PC, USB, Hub, CPU, SLED and VOA units, which are responsible for generating light beams, managing the overall operation of the OCT and, later, collecting and processing data to a form usable for diagnostics. This occurs in a manner typical of the solutions in this field, so the operating details of these elements are not described herein more specifically, as they are well known to a person skilled in the art.

A generated light beam with appropriate initial parameters (wavelength, light intensity, etc.) first reaches the optical divider/coupler 17, where it is divided into two beams: the object beam and the reference beam.

The reference beam is directed to the optical path of the reference beam 16, while the object beam is directed to the optical path of the object beam 22, where, by passing through the elements of the autofocus system 14, scanners XY and other optical elements, it finally reaches the tested eye and then returns the same way to the optical coupler 17. There, both beams - the reference beam and the object beam - interfere with each other and are directed to the S_OCT spectrometer. Optionally, an additional lens (see 23 in Fig. 12) can also be put into the object optical path, which changes the character of bending the beams between the optical lens and the tested eye, i.e., the structures of the anterior eye chamber (the cornea, the lens, etc.). In the anterior mode, the scanning beam is convergent, while without it, it is parallel and illustrates the structures of the posterior section of the eye (the retina, the optic nerve disc, etc.).

Although many improvements have been made during years in the optical elements described above, composing the optical paths of the reference and object beams, this invention concerns modifications made in a different part of OCTs; even in the OCT spectrometer itself, where a beam of light beams delivered through the optical fibre output 1 reaches first the collimator 2, then the diffraction element 3, and then, having passed through the optical lens, it finally reaches the appropriate detector 5.

The objective of this invention is achieved by a modification made in the area of the OCT spectrometer system, in particular by modifications related to the optical lens 4 and the diffraction element 3. However, there may be many types of modifications; below we present some of them without limitation.

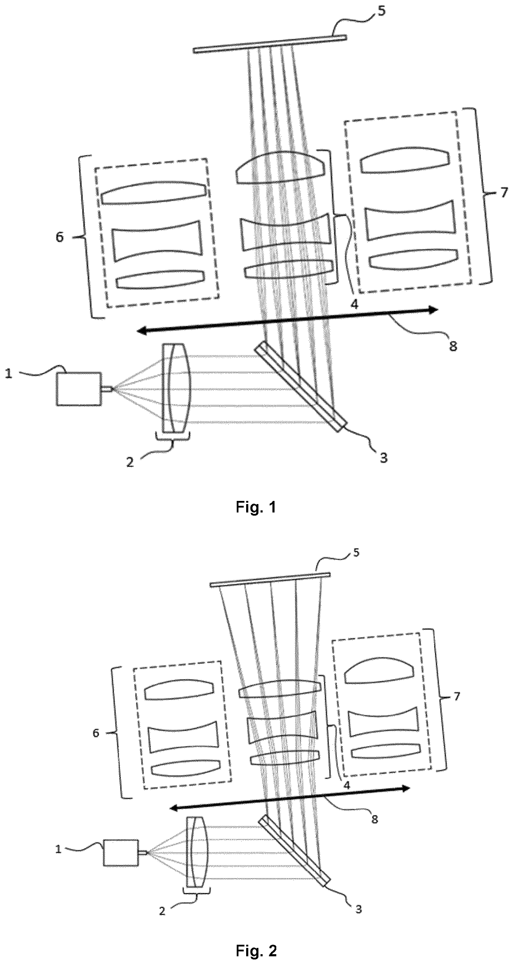

### Example 1: three sets of optical lenses (Fig. 1 and Fig. 2)

Figs. 1 and 2 show the first variant of modifying the structure of OCT scanners according to the invention, enabling a change in the depth of the imaging window.

The example solution presents the internal structure of an OCT scanner with three sets of lenses composing three optical lenses 4, 6 and 7, each of them including three lenses that are located at fixed distances to one another. The sets of lenses are used as the optical lenses 4, 6 and 7 and are installed in a revolving handle that allows a particular device from the selected optical lenses 4, 6 or 7 to be put into the optical path.

The optical lenses 4, 6 and 7 differ from one another in focal length; we can choose between short, medium and long focal length.

The medium focal length is used in a standard eye with no pathological changes, so in many cases we can assume that this is the basic optical lens used at the beginning of an eye test.

If a pathological change is found in the eye, an appropriate different optical lens is set in the device by means of the revolving handle, according to the nature of the change, to enable better imaging.

If the change goes beyond the device's imaging area along the z axis, then an optical lens with a longer focal length is used that enables a higher imaging window than with the medium optical lens and, consequently, can cover a wider depth range at the z axis of the eye. At that axis, the image is smaller than that obtained with the use of the medium optical lens (the image from the higher imaging window is "compressed" so that it can fit into the height of the imaging window of the medium optical lens).

By contrast, if the change is small and requires magnification in the image, the optical lens with the shortest focal length is put into the device's optical path. The shortest focal length provides the shortest imaging window, so we achieve a narrower depth range presented by the spectrometer. This allows us to magnify the change in the image (the image from the lower window is "extended" so that it fits into the height of the imaging window of the medium optical lens).

In one preferable embodiment, the OCT spectrometer system with several optical lenses 4, 6, 7 can be implemented by means of a spectrometer with only two optical lenses that will serve the same functions as in a system with the three optical lenses 4, 6, 7, the difference being that one of them will have a longer focal length (which will make it possible to achieve a higher imaging window) while the other one will have a shorter focal length (which will make it possible to achieve a lower imaging window).

Such a preferable system is implemented by, for example, introducing two lenses, with the spectrometer fitted with the first optical lens having the following parameters:
- Focal length of the optical lens imaging spectrum on the camera: 68.946 mm,
- Spectral width fitting on a ruler: 108.269 nm,
- Imaging depth in the air: 3.403 mm,
- Spectrometer resolution in the air: 6.376 µm.
The spectrometer fitted with the second optical lens has the following parameters:
- Focal length of the optical lens imaging spectrum on the camera: 163.500 mm,
- Spectral width fitting on a ruler: 45.656 nm,
- Imaging depth in the air: 8.097 mm,
- Spectrometer resolution in the air: 6.376 µm.

This embodiment of the invention has a benefit of the mechanical (revolving) system for rotating/replacing the optical lenses 4, 6, 7 being able to provide high diversity of the optical lenses 4, 6, 7, both in terms of their quantity and specified imaging heights.

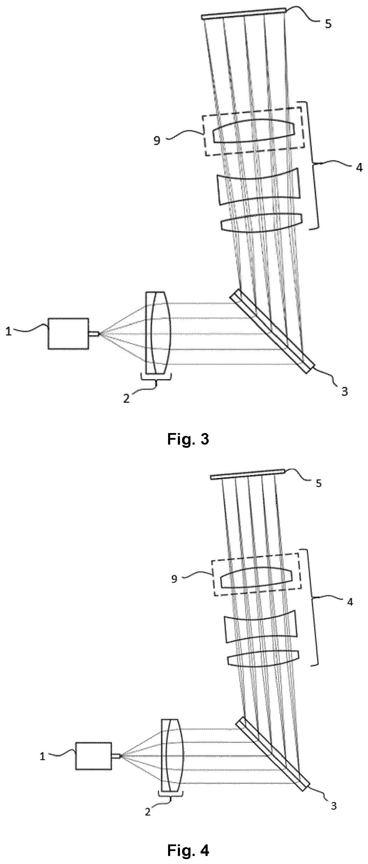

### Example 2: multifocal lens (Fig. 3 and Fig. 4)

Figs. 3 and 4 show another variant of modifying the OCT scanners according to the invention, wherein the set of replaceable optical lenses 4, 6, 7 for changing the height of the imaging window is substituted with a single movable multifocal lens 9.

This example solution presents a device with a single set of lenses used as an optical lens 4 that includes one multifocal lens 9 that is positioned at fixed distances from the other lens of the optical lens 4.

Preferably, the multifocal lens 9 consists of a polymer membrane and an optical fluid that is located between the membrane and the front wall of the lens 9. When affected by a ring having the diameter of the lens 9, the shape and the focal length of the lens 9 can be modified by the ring pressing against the external part of the membrane or by pumping liquid into the lens 9.

A change in the focal length of the lens 9 modifies the focal length of the entire optical lens 4, which makes it possible not only to achieve the medium height of the imaging window but also a higher or lower imaging window.

In practice, window imaging preferably begins at the medium imaging height.

If a pathological change is found that goes beyond the imaging area at the z axis, the focal length of the multifocal lens 9 is extended, that is, the lens 9 is flattened with the ring (the radius of the rear surface is extended). This way, we achieve a higher imaging window that allows for imaging a wider scope of depths in a single image (the image is "compressed" to the height of the imaging window obtained by means of the medium curvature radius of the rear surface of the lens 9). This means that the image will be reduced in size at the z axis, and the change will be fully visible.

If the pathological change is small and must be enlarged at the z axis, the focal length of the multifocal lens 9 is shortened, and the lens 9 is made convex by pumping an optical fluid inside it (the radius of the rear surface of that lens 9 is reduced). This way, we obtain a lower imaging window that allows achieving a narrower depth range in a single image (the image is "extended" to the height of the imaging window obtained by means of the medium curvature radius of the rear surface of the lens 9), which in turn means that the image of the pathological change is enlarged at the z axis, which makes it possible to diagnose the change more precisely.

In another example of the implementation of this embodiment, the optical system can be implemented by means of an OCT spectrometer with one conventional lens and one multifocal lens 9, for example ML-20-37 by Optotune (with only two lenses within the lens 4). Both lenses are permanently positioned at the same distance from each other and from the diffraction grade 3. The varifocal (multifocal) lens 9 can change the focus of the entire optical lens 4 so that, in one configuration, it will have a higher focal length (which will make it possible to achieve a higher imaging window), and in the other configuration, it will have a lower focal length (which will make it possible to achieve a lower imaging window).

In one preferred embodiment, the system described above is implemented by various settings of the multifocal lens 9, the spectrometer with the first setting of the multifocal lens 9 having the following parameters:
- Focal length of the optical lens imaging spectrum on the camera: 59.405 mm,
- Spectral width fitting on a ruler: 115.000 nm,
- Imaging depth in the air: 3.153 mm,
- Spectrometer resolution in the air: 6.376 µm.

The spectrometer with the other setting of the multifocal lens 9 has the following parameters:
- Focal length of the optical lens imaging spectrum on the camera: 67.757 mm,
- Spectral width fitting on a ruler: 104.000 nm,
- Imaging depth in the air: 3.502 mm,
- Spectrometer resolution in the air: 6.376 µm.

Compared to example 1, this embodiment of the invention has a benefit of the optical lens 4 with the multifocal lens 9 taking up less space (even if we take into account the means for adjusting the curvature of the multifocal lens 9) than the revolving system of several optical lenses 4, 6, 7.

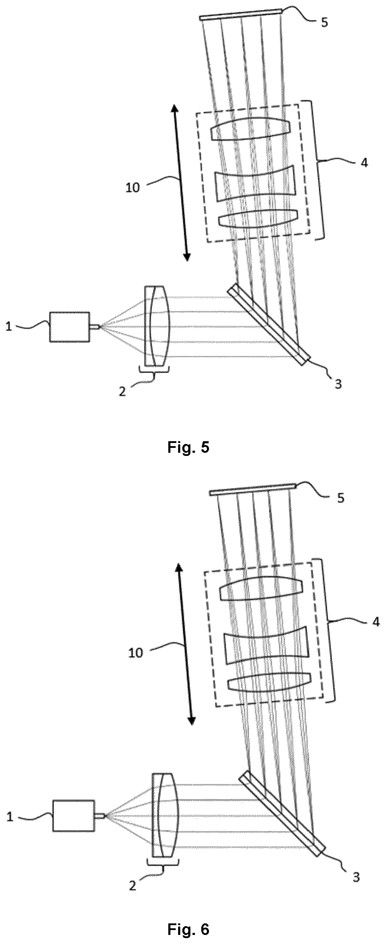

### Example 3: optical lens shift (Fig. 5 and Fig. 6)

Figs. 5 and 6 show another variant of modifying the OCT scanners according to the invention, wherein the height of the imaging window is changed by linear/axial shifting (see arrow 10 in Figs. 5 and 6) of the entire optical lens 4.

The example solution shows a device using one set of lenses as an optical lens 4. All lenses of the optical lens 4 are located at fixed distances from one another. The set is installed on a motorised table that enables shifting all the lenses at the same time along the device's optical axis, thus moving the optical lens 4 closer to or farther from the detector 5. By moving the optical lens 4 closer to the detector 5, we obtain a lower imaging window, and by moving it away, we obtain a higher imaging window.

If the pathological change goes beyond the imaged area at the z axis, the table with the optical lens 4 should be moved away from the detector 5 so that the imaging window is high enough to accommodate the whole change in the obtained image. A higher imaging window means that a wider depth range will be imaged, so the image will be reduced in size compared to the image obtained with the use of the optical lens 4 in the medium setting (the image will be "compressed" to the height of the imaging window obtained with the standard setting of the table with the optical lens 4) and the whole change will be visible in the image.

If the change requires to be enlarged in the image for more precise diagnostics, the table with the optical lens 4 should be moved closer to the detector 5. A lower imaging window means that a narrower depth range will be imaged, so the image will be enlarged at the z axis compared to the image obtained with the optical lens 4 in the medium setting (the image is "extended" to the height of the imaging window obtained with the medium setting of the table with the optical lens 4).

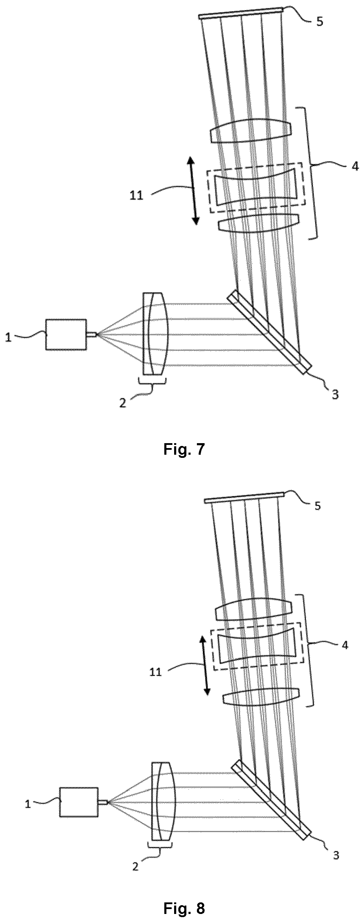

### Example 4: shift of one of the lenses of the optical lens (Fig. 7 and Fig. 8)

Figs. 5 and 6 show yet another variant of modifying the OCT scanners according to the invention, wherein the height of the imaging window is changed by linear/axial shifting one of the lenses composing the optical lens 4.

The example solution shows a device using one set of lenses as an optical lens 4. Two of the three lenses are positioned at a fixed distance from each other. The third lens is installed on a motorised table that allows shifting that lens relative to the other lenses and the detector 5 along the device's optical path. If we move the lens closer to or farther from the detector 5, we make it possible to change the focal length of the whole optical lens 4, which in turn changes the height of the device's imaging window.

By moving the lens closer to the detector 5, a lower focal length of the optical lens 4 will be obtained, and the imaging window will be lower, so the image will show a narrower depth range. In this way it is possible to magnify the pathological change in the obtained image, as the image will be "extended" to the height of the imaging window produced by the lens in medium setting relative to the detector 5. Magnifying the image in turn makes it possible to diagnose a small pathological change with more precision, which would be difficult using the medium setting of the lens relative to the detector 5.

By moving the lens away from the detector 5, a higher focal length will be obtained, and the imaging window will be higher, so the image will show a wider depth range. In this way it is possible to image a larger area into the eye. By using a higher imaging window for large changes that go beyond the imaging window in the medium setting of the lens relative to the detector 5, the image will be "compressed" to the height of the imaging window obtained with the lens in the medium setting. Reducing the image in size will in turn will make it possible to depict the entire pathological change in one image.

In one preferred embodiment, the system described above is implemented by various settings of one of the lenses of the optical lens, the spectrometer with the first setting of the lens relative to the detector 5 having the following parameters:
- Focal length of the optical lens imaging spectrum on the camera: 75.663 mm,
- Spectral width fitting on a ruler: 88.000 nm,
- Imaging depth in the air: 4.173 mm,
- Spectrometer resolution in the air: 6.376 µm.

The spectrometer with the other setting of the lens relative to the detector 5 has the following parameters:
- Focal length of the optical lens imaging spectrum on the camera: 152.997 mm,
- Spectral width fitting on a ruler: 46.000 nm,
- Imaging depth in the air: 8.036 mm,
- Spectrometer resolution in the air: 6.376 µm.

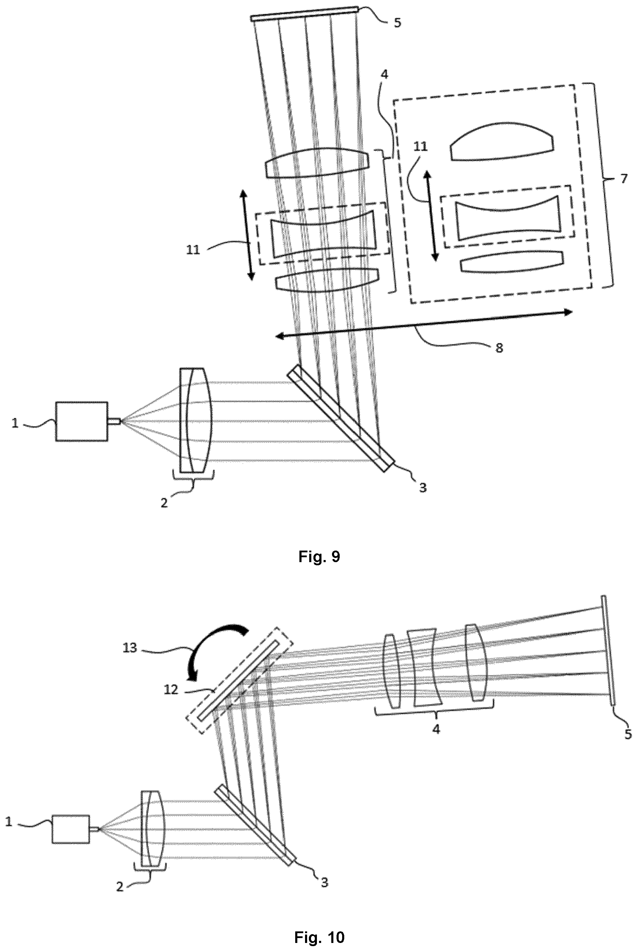

### Example 5: two optical lenses with a shift of one of the lenses (Fig. 9)

Fig. 9 shows yet another variant of modification to the OCT scanners according to the invention, wherein the height of the imaging window is changed by means of a combination of the examples described above, i.e., by providing two replaceable optical lenses 4 and 7, both fitted with means for linear/axial shifting of one of the lenses (along the direction of the axial shift 11 of the lens in the optical lenses 4 and 7).

The example solution shows a device using two sets of lenses as optical lenses 4 and 7. Each of them has one lens installed on a table that allows shifting them along the device's optical path. Each of the optical lenses 4 and 7 can change its focal length.

Each of the optical lenses 4 and 7 works according to the principle described in example 4. Optical lenses 4 and 7 are installed on a drum that allows putting one of them into the device's optical axis. The use of two separate optical lenses 4 and 7 enables more significant changes in eye imaging depth, so bigger differences in imaging heights. By choosing one of the optical lenses 4 or 7, it is possible to choose between a higher or lower imaging window, which effectively means a choice between a reduced or enlarged image.

For an optical lens 4, 7 with a higher focal length that can produce a lower imaging window, if we move the lens farther from the detector 5, we can obtain imaging that corresponds to the medium optical lens 4 in example 1. As we move the lens closer to the detector 5, we will obtain gradually lower imaging windows and thus observe an image with higher magnification rates; this allows us to largely adjust the window size to small changes that require magnification for more precise diagnostics.

Conversely, for an optical lens 4, 7 with a lower focal length that can produce a higher imaging window, if we move the lens closer to the detector 5, we can obtain imaging that corresponds to the medium lens in example 1. As we move the lens away from the detector 5, we will obtain gradually higher imaging windows and thus the image observed will be gradually reduced in size, which allows us to largely adjust the window size to big pathological changes that require magnification for more precise diagnostics. Therefore, as we move the lens away, a single image will cover increasingly bigger pathological changes.

In one preferred embodiment, the system described above is implemented by introducing two optical lenses 4 and 7 with one movable lens. In addition to the biggest and smallest imaging windows, we can also make intermediate settings. The spectrometer fitted with the first optical lens 4 and the lens in the first setting (the highest imaging window) has the following parameters:
- Focal length of the optical lens imaging spectrum on the camera: 152.997 mm,
- Spectral width fitting on a ruler: 46 nm,
- Imaging depth in the air: 8.036 mm,
- Spectrometer resolution in the air: 6.376 µm.

The spectrometer fitted with the first optical lens 4 and the lens in the second setting (high imaging window) has the following parameters:
- Focal length of the optical lens imaging spectrum on the camera: 135.232 mm,
- Spectral width fitting on a ruler: 50.000 nm,
- Imaging depth in the air: 7.392 mm,
- Spectrometer resolution in the air: 6.376 µm.

The spectrometer fitted with the second optical lens 7 and the lens in the fourth setting (low imaging window) has the following parameters:
- Focal length of the optical lens imaging spectrum on the camera: 65.056 mm,
- Spectral width fitting on a ruler: 101.000 nm,
- Imaging depth in the air: 3.654 mm,
- Spectrometer resolution in the air: 6.376 µm.
The spectrometer fitted with the second optical lens 7 and the lens in the third setting (the lowest imaging window) has the following parameters:
- Focal length of the optical lens imaging spectrum on the camera: 63.932 mm,
- Spectral width fitting on a ruler: 110.000 nm,
- Imaging depth in the air: 3.341 mm,
- Spectrometer resolution in the air: 6.376 µm.

### Example 6: additional rotary diffraction element (Fig. 10)

Fig. 10 shows yet another variant of modifying the OCT scanners according to the invention, wherein the height of the imaging window is changed by placing the second diffraction element 12 in the optical path (between the first diffraction element 3 and the optical lens 4) together with means for modifying its angular position relative to the first diffraction element 3 and the detector 5, by rotating it (see arrow 13 in Fig. 10).

The example solution presents a device using two diffraction grids as diffraction elements 3 and 12 that are located in the optical path upstream of the optical lens 4. The first of them is a transmission grid 3. The beam is refracted in the material of the grid 3, and the diffracted waves go out on the other side of the diffraction element 3. The other grid 12 is a reflection grid 12 that reflects the beam at its surface.

In this embodiment, the reflection grid 12 is installed on a motor that allows changing the angle at which the beam will fall on its surface. This allows us to change the height of the imaging window, so the magnification of the image observed by the device.

By rotating the grid 12 to one side, it is possible to obtain a lower imaging window The produced image will be "compressed" to the height of the imaging window of the grid that is not rotated. The eye structures in that image will be increased at the z axis, so for small pathological changes it will be possible to carry out more precise diagnostics.

Conversely, by rotating the grid 12 to the other side, it is possible to obtain a higher imaging window. The produced image will be "extended" to the height of the imaging window of the grid that is not rotated. The image will present the structures reduced in size, so it will be possible to observe big pathological changes going beyond the depth range of the imaging window for the grid that is not rotated.

Another such system can be implemented by the introduction of two transmission grids. The first of them is characterised by an incidence angle of 49° and a density of 1800 l/mm. The other is characterised by an incidence angle of 49° and a density of 1765 l/mm. The first grid can rotate by 2°.

A spectrometer with grid 12 rotated by 1° to the left (lower imaging window) has the following parameters:
- Focal length of the optical lens imaging spectrum on the camera: 71.764 mm,
- Spectral width fitting on a ruler: 48.000 nm,
- Imaging depth in the air: 7.574 mm,
- Spectrometer resolution in the air: 6.376 µm.

A spectrometer with grid 12 rotated by 1° to the right (higher imaging window) has the following parameters:
- Focal length of the optical lens imaging spectrum on the camera: 71.764 mm,
- Spectral width fitting on a ruler: 46.000 nm,
- Imaging depth in the air: 8.036 mm,
- Spectrometer resolution in the air: 6.376 µm.

### Example 7: two rotary diffraction elements (Fig. 11)

Fig. 11 shows yet another variant of modifying the OCT scanners according to the invention, being more extensive compared to the variant described in example 6, wherein the height of the imaging window is changed by placing a second diffraction element 12 in the optical path (between the first diffraction element 3 and the optical lens 4) together with means for modifying the angular position of both these diffraction elements 3 and 12 (see arrow 13 in Fig. 11).

The example solution presents a device using two diffraction grids as diffraction elements 3 and 12 as in example 6, the difference being that, in this case, both grids 3 and 12 are installed on motors that make it possible to change the angle at which the beam will fall on the surface of the grids 3 and 12. This makes it possible to change the height of the imaging window to a larger extent than in example 6.

In this case, a precise and mutual rotation of both grids 3 and 12 allows us to set the height of the imaging window more precisely for best possible imaging of the pathological change.

Another such system can be implemented by the introduction of two transmission grids 3 and 12. The first of them is characterised by an incidence angle of 49° and a density of 1800 l/mm. The other is characterised by an incidence angle of 49° and a density of 1765 l/mm. The first of the grids 3 can rotate by 2°, and the other 12 can rotate by 4°. A spectrometer with the first grid 3 rotated by 1° to the right and the other grid 12 rotated by 2° to the right (higher imaging window) has the following parameters:
- Focal length of the optical lens imaging spectrum on the camera: 71.764 mm,
- Spectral width fitting on a ruler: 44.000 nm,
- Imaging depth in the air: 8.128 mm,
- Spectrometer resolution in the air: 6.376 µm.

A spectrometer with the first grid 3 rotated by 1° to the right and the other grid 12 rotated by 1° to the right (medium imaging window) has the following parameters:
- Focal length of the optical lens imaging spectrum on the camera: 71.764 mm,
- Spectral width fitting on a ruler: 45.000 nm,
- Imaging depth in the air: 8.051 mm,
- Spectrometer resolution in the air: 6.376 µm.

A spectrometer with the first grid 3 rotated by 1° to the left and the other grid 12 rotated by 1° to the right (lower imaging window) has the following parameters:
- Focal length of the optical lens imaging spectrum on the camera: 71.764 mm,
- Spectral width fitting on a ruler: 47 nm,
- Imaging depth in the air: 7.562 mm,
- Spectrometer resolution in the air: 6.376 µm.

Considering the presented numerous ways to attain the objective of this invention, it becomes understandable that this description can only present some of the many preferred and possible embodiments. The combinations described in the examples do not exhaust all all possible options for modifying the optical system of an OCT scanner to provide particular, desirable heights of the imaging window.

A person skilled in in the art would be able to develop a range of other modifications that would remain in the spirit of this disclosure, for example to ensure movability of individual lenses or the entire optical lens 4 and at the same time to provide an additional, rotatable diffraction element 12, or to use a deformable multifocal lens 9 as a movable lens in the optical lens 4.

Each variant can be adequately adapted to meet particular expectations, for instance to reduce the time of measurement, provide significantly higher measuring accuracy, allow for making measurements of "regular quality" for a given height of the imaging window, but provide much "sharper" images for imaging pathological changes in the eye at particular, predefined depths in the eye.

The use of this invention enables us to considerably increase the detection capability of eye OCTs while maintaining user safety, as there are no important modifications compared to the already existing devices regarding the optical path that drives the light beam towards the eye of the tested person, but we only change the structure of the OCT scanner (so the "last" part of OCT scanners from the point of view of the path of emitted light beams).

Furthermore, by integrating the proposed modifications, the paths of the optical OCT scanner, upon introducing/removing an additional lens to/from the optical path for switching the operating mode of the OCT between the posterior and the anterior part of the eye (see arrow 23 in Fig. 12), it is possible to improve the quality and accuracy of imaging big pathological changes not only in the area of the retina/optic nerve but also in the area of the front eye chamber (lens, cornea, etc.), which improves the diagnostic capability of OCTs even further.

## Claims

1. A device for measuring the characteristics of the human eye in the OCT technology, used for measuring eye properties, that has the following components within the optical path of the light beam:
- a light beam source with a low time coherence,
- a light beamsplitter used for dividing a single light beam coming from the light beam source into two and directing them towards the tested eye and the reference path and for directing the light beams that return from the reference path and from the tested eye, which enables the interference of those returning light beams and directing them further to the appropriate spectrometer,
- optical elements of the reference path and optical elements for directing the light onto the tested eye,
- an appropriate spectrometer comprising:
- a collimator (2),
- a diffraction element (3),
- an optical lens (4),
- a detector (5),
**characterised in that** the device is provided with additional optical means configured and suited for adjusting the spectrometer's imaging window by adjusting the spectral range of the wavelength recorded by the detector, which means are located within the spectrometer.

2. The device according to claim 1, wherein the additional optical means are selected from a group including a set of replaceable optical lenses (4, 6, 7), a deformable multifocal lens (9), means for shifting the optical lens (4) relative to the detector (5), means for shifting one of the lenses in the optical lens (4), and an additional rotating diffraction element (12), or a combination thereof.

3. The device according to claim 2, wherein the additional optical means have the form of a set of optical lenses (4, 6, 7) that replace a single optical lens (4), each of which is suitable for providing a different imaging window height.

4. The device according to claim 3 provided with two to five sets of optical lenses (4, 6, 7).

5. The device according to claim 4 provided with three sets of optical lenses (4, 6, 7).

6. The device according to claim 2, wherein the additional optical means have the form of a deformable multifocal lens (9) being part of the spectrometer's optical lens (4), which is accompanied by means for deforming the multifocal lens.

7. The device according to claim 2, wherein the additional optical means have the form of means for shifting the position of the optical lens (4) relative to the detector (5).

8. The device according to claim 2, wherein the additional optical means have the form of means for shifting one of the lenses of the optical lens (4).

9. The device according to claims 4 and 8, wherein the additional optical means have the form of two sets of optical lenses (4, 7), each of which is provided with means for shifting one of the lenses.

10. The device according to claim 2, wherein the additional optical means have the form of the second diffraction element (12) located between the diffraction element (3) and the optical lens (4), which the second diffraction element (12) is provided with means for shifting its position by changing its position angle.

11. The device according to claim 10, wherein the first diffraction element (3) is also provided with means for changing its position by changing its position angle.

12. The device according to any of previous claims 2 to 11, additionally fitted with a movable focal lens (23) for switching the light beam focusing area between the posterior part of the eye (i.e., the area of the retina) and the anterior chamber of the eye (i.e., the area of the cornea and lens).

## Amended claims

### Amended claims in accordance with Rule 137(2) EPC.

1. A device for measuring the characteristics of the human eye in the OCT technology, used for measuring eye properties, that has the following components within the optical path of the light beam:
- a light beam source with a low time coherence,
- a light beamsplitter used for dividing a single light beam coming from the light beam source into two and directing them towards the tested eye and the reference path and for directing the light beams that return from the reference path and from the tested eye, which enables the interference of those returning light beams and directing them further to the appropriate spectrometer,
- optical elements of the reference path and optical elements for directing the light onto the tested eye,
- an appropriate spectrometer comprising:
- a collimator (2),
- a diffraction element (3),
- an optical lens (4),
- a detector (5),
wherein the device is provided with additional optical means configured and suited for adjusting the spectrometer's imaging window by adjusting the spectral range of the wavelength recorded by the detector, which means are located within the spectrometer,
**characterized in that** the additional optical means are selected from a group including a set of replaceable optical lenses (4, 6, 7), a deformable multifocal lens (9), means for shifting one of the lenses in the optical lens (4), and an additional rotating diffraction element (12), or a combination thereof.

2. The device according to claim 1, wherein the additional optical means have the form of a set of optical lenses (4, 6, 7) that replace a single optical lens (4), each of which is suitable for providing a different imaging window height.

3. The device according to claim 2 provided with two to five sets of optical lenses (4, 6, 7).

4. The device according to claim 3 provided with three sets of optical lenses (4, 6, 7).

5. The device according to claim 1, wherein the additional optical means have the form of a deformable multifocal lens (9) being part of the spectrometer's optical lens (4), which is accompanied by means for deforming the multifocal lens.

6. The device according to claim 1, wherein the additional optical means have the form of means for shifting one of the lenses of the optical lens (4).

7. The device according to claims 3 and 6, wherein the additional optical means have the form of two sets of optical lenses (4, 7), each of which is provided with means for shifting one of the lenses.

8. The device according to claim 1, wherein the additional optical means have the form of the second diffraction element (12) located between the diffraction element (3) and the optical lens (4), which the second diffraction element (12) is provided with means for shifting its position by changing its position angle.

9. The device according to claim 8, wherein the first diffraction element (3) is also provided with means for changing its position by changing its position angle.

10. The device according to any of previous claims 1 to 9, additionally fitted with a movable focal lens (23) for switching the light beam focusing area between the posterior part of the eye (i.e., the area of the retina) and the anterior chamber of the eye (i.e., the area of the cornea and lens).
